# EUROPEAN PATENT SPECIFICATION

(11) **EP 0 666 757 B1**
(45) Date of publication and mention of the grant of the patent: **11.03.1998**
(21) Application number: 93921769.1
(22) Date of filing: 04.10.1993
(51) Int. Cl.: A61K 38/00, A61K 38/46, A61K 39/39

(54) **ANTI-INFLAMMATORY, TOLEROGENIC AND IMMUNOSTIMULATORY PROPERTIES OF CARBOHYDRATE BINDING-PROTEINS**
ENTZÜNDUNGSHEMMENDE TOLEROGENE UND INMONOINHIBITORISCHE EIGENSCHAFTEN VON KARBOHYHYDRATE BINDENDEN PEPTIDEN
CARACTERISTIQUES ANTI-INFLAMMATOIRES, TOLEROGENES ET IMMUNO-STIMULANTES DE PROTEINES DE LIAISON D'HYDRATE DE CARBONE

(30) Priority: 02.10.1992 US 956043
(43) Date of publication of application: 16.08.1995
(73) Proprietor: ALBERTA RESEARCH COUNCIL, Edmonton Alberta T6H 5X2 (CA)
(72) Inventor: SMITH, Richard, Edmonton, Alberta T6R 2A6 (CA); HEERZE, Louis, D., Edmonton, Alberta T6E 2N1 (CA); ARMSTRONG, Glen, D., Edmonton, Alberta T6C 1P6 (CA)
(74) Representative: Nash, David Allan
(86) International application number: PCT/CA93/00414
(87) International publication number: WO 94/07516

(56) References cited:
- EP-A- 0 173 092
- EP-A- 0 338 566
- WO-A-93/18782

## Description

### BACKGROUND OF THE INVENTION

### 1. Field of the Invention.

The present invention is directed to methods for enhancing or inhibiting immune responses or cellular interactions by the administration of carbohydrate binding proteins. In particular, the present invention is directed to methods for the suppression of inflammatory responses, induction of tolerance to antigens, modulation of the induction of immune responses to antigens, and the inhibition or enhancement of cell adhesion by the administration of carbohydrate binding proteins.

### 2. References.

The following references are cited in this application as superscript numbers at the relevant portion of the application:
1. Brandley et al., *J. Leukocyte Biol.*, 40:97-111 (1986).
2. Jacobson, *Developmental Neurobiology*, New York, Plenum Press p. 5-25, (1978).
3. Trinkaus, *Cells into Organs*, Englewood Cliffs, N.J., Prentice Hall, p. 44-68, (1984).
4. Frazier et al., Annu. Rev. Biochem., 48:491 (1979).
5. Glaser, *Mediator of Developmental Processes* (Substency, S. And Wessels, N.K., Eds.) New York, Academic Press, p. 79 (1980).
6. Paulson, *In "The Receptors"*, Vol. II (Comm., P.M., Ed.), New York Academic Press, p. 131 (1985).
7. Sharon, Lectin-Like Bacterial Adherence to Animal Cells. In "Attachment of Microorganisms to the Gut Mucosa" (Boeheker, E.D., Ed.), Boca Raton, Florida CRC Press, p. 129 (1984).
8. Wassarman, Fertilization. In "Cell Interactions and Development: Molecular Mechanisms" (Yamada, K.M., Ed.), New York, John Wiley and Sons, p. 1 (1983).
9. Schwartz et al., *Immunol. Rev.*, 40:153 (1978).
10. Coutinho et al., *Immunol. Rev.*, 78:211 (1984).
11. Hoffmann et al., Eds., *Membranes in Growth and Development*, New York, Alan R. Liss, p. 429-442, (1982).
12. Galeotti et al., Eds., *Membranes in Tumor Growth*, Amsterdam, Elsevier, p. 77-81, (1982).
13. Nicolson et al., *Invas. Metas.*, 5:144 (1985).
14. Aplin et al., *Biochim. Biophys. Acta* 694:375 (1982).
15. Barondes, Developmentally Regulated Lectins. In "Cell Interactions and Development: Molecular Mechanisms" (Yamada, D.M., Ed.) New York, John Wiley and Sons, p. 185 (1983).
16. Monisigny, M., Ed., *Biol. Cell*, 51 (Special Issue), 113, 1984.
17. Springe et al., *Nature*, 349:196-197 (1991).
18. Lowe et al., *Cell*, 63:475-485 (1990).
19. Phillips et al., *Science*, in press (1990).
20. Walz et al., *Science*, 250:1132 et seq. (1990).
21. Larsen et al., *Cell*, 63:467-474 (1990).
22. Bevilacqua et al., Endothelial-Leukocyte Adhesion Molecule - 1 (ELAM-1): A Vascular SELECTIN That Regulates Inflammation. In "Cellular and Molecular Mechanisms of Inflammation" Vol. 2, *Academic Press*, p. 1-13 (1991).
23. McEver, Leukocyte Interactions Mediated by GMP-140. In "Cellular and Molecular Mechanisms of Inflammation", Vol. 2, *Academic Press*, p. 15-29 (1991).
24. Larsen et al., *J. Biol. Chem.*, 267:11104-11110 (1992).
25. Heerze et al., *Biochem and Biophys. Res. Comm.* 172:1224-1229 (1990).
26. Paulson et al., *International Publication No. WO91*/*19502* (1991).
27. Ippolito et al., U.S. Serial No. 07/714,161 filed June 10, 1991.
28. Venot et al., U.S. Serial No. 07/889,017, filed May 26, 1992.
29. McEver, *International Publication No. WO92*/*01718* (1992).
30. Heerze et al., *Biochem and Biophys. Res. Comm.* 179:1464-1469 (1991).
31. Pearce-Pratt et al., *J. Imm. Methods* 140:159-165 (1991).
32. Smith et al., *Cell. Imm.* 89:20-29 (1984).
33. Munoz, Action of Pertussigen (Pertussis Toxin) on the Host Immune System. In "Pathogenesis and Immunity in Pertussis", John Wiley & Sons Ltd. p. 173-192 (1988).
34. Reuter et al, *Glycoconjugates* 5:133-135 (1988).

The disclosure of all publications, patents and patent applications are herein incorporated by reference in their entirety.

### 3. State of the Art.

Important processes involving mammalian cells, such as growth, locomotion, morphological development, and differentiation are partially controlled by extracellular signals acting upon the cells' surfaces¹⁻³. While some external stimuli reach the cell via extracellular fluids, other signals are received from neighboring or approaching cell surfaces and exert their effects through direct cell-cell contact^{4,5}.

Evidence suggests that specific cell-surface receptors can "sense" a molecular signal of an apposing cell via specific binding, and biochemical mechanisms exist to translate that binding into a cellular response. For example, complex cell-surface interactions are believed to help direct processes such as binding of pathogens to target tissues^{6,7}, sperm-egg binding⁸, interactions among cells in the immune system^{9,10}, and recognition of cells during embryonic development¹¹. In addition, defects in cell-cell recognition are thought to underlie the uncontrolled cell growth and motility which characterize neoplastic transformation and metastasis^{12,13}.

Other evidence suggests that cell-recognition processes are mediated by carbohydrate chains or glycan portions of glycoconjugates^{4,14-16}. For example, the binding of the surface glycoconjugates of one cell to the complementary carbohydrate-binding proteins (lectins) on another cell can result in the initiation of a specific interaction.

One important group of carbohydrate-binding proteins are selectins (LEC-CAM) proteins (Lectin + EGF + complementary Regulatory Domain-Cell Adhesion Molecules). These or functionally similar proteins or lectins may play a critical role in immune responses (including inflammatory responses) through mediation of cell-cell contact and through extra-vasation of leucocytes¹⁷⁻²². Specific carbohydrate ligands have been identified as part of the putative receptor structures for selectins proteins and other lectins ¹⁷⁻²⁵. The structures identified include α-2,6 and α-2,3 sialic acid structures.

The use of oligosaccharides and derivatives thereof for controlling inflammation, immunosuppression, and inducing tolerance to an antigen by interacting with selectin proteins and/or other lectins has been disclosed ²⁶⁻²⁸. Peptides derived from the selectin GMP-140 which inhibit binding of GMP-140 and other selectins have also been described ²⁹.

### SUMMARY OF THE INVENTION

The present invention is directed to the discovery that certain carbohydrate binding proteins, e.g., lectins, when administered to a mammal enhance or inhibit specific immune responses and cellular interactions. In particular, the present invention is directed to the discovery that carbohydrate binding proteins may be administered to a mammal in order to inhibit inflammatory responses, modulate the induction of an immune response to an antigen, induce long term tolerance to an antigen, and suppress or enhance cell adhesion.

The present invention is particularly directed to the discovery that carbohydrate binding proteins capable of binding terminal sialic acid groups or molecules containing such terminal sialic acid groups may be administered to a mammal as a means for inhibiting or enhancing particular immune responses or cell adhesion. The present invention is more particularly directed to the discovery that lectins capable of binding terminal linked α-2,6 sialic acid structures and/or α-2,3 sialic acid structures may be administered to a mammal in order to enhance or inhibit various immune responses, including inflammatory responses, modulation of the induction of the immune response to an antigen, and induction of long term tolerance to an antigen. Additionally, the present invention is directed to the discovery that carbohydrate binding proteins capable of binding α-2,6 sialic acid structures and/or α-2,3 sialic acid structures may be administered to a mammal in order to suppress or enhance cell adhesion.

Accordingly, in one of its aspects, the present invention is directed to the use of at least one carbohydrate binding protein or fragment or derivative thereof capable of binding terminal α-2,6 sialic acid structures and/or α-2,3 sialic acid structures or molecules comprising such sialic acid structures for the preparation of a pharmaceutical composition for suppressing an inflammatory response in a mammal.

In another one of its aspects, the present invention is directed to the use of at least one carbohydrate binding protein, e.g., a lectin, or a fragment or derivative thereof capable of binding terminal α-2,6 sialic acid structures and/or α-2,3 sialic acid structures for the preparation of a pharmaceutical composition for modulating the induction of an immune response to an antigen whereby the mammal is immunized with an antigen in combination with the protein.

In another one of its aspects, the present invention is directed to the use of at least one carbohydrate binding protein, e.g., a lectin, or a fragment or derivative thereof capable of binding terminal α-2,6 sialic acid structures and/or α-2,3 sialic acid structures for the preparation of a pharmaceutical composition for inducing tolerance to an antigen in a mammal whereby the protein is administered to a mammal which has previously been exposed to an antigen.

In still another one of its aspects, the present invention is directed to the use of at least one carbohydrate binding protein, e.g, a lectin, or a fragment or derivative thereof capable of binding terminal α-2,6 sialic acid structures and/or α-2,3 sialic acid structures for the preparation of a pharmaceutical composition for treating or inhibiting metastasis.

In yet another one of its aspects, the present invention is directed to the use of one or more carbohydrate binding proteins, e.g., lectins, or a fragment or derivative thereof capable of binding terminal α-2,6 sialic acid structures and/or α-2,3 sialic acid structures for the preparation of a pharmaceutical composition for treating or inhibiting lung inflammation or a lung inflammatory disorder.

### BRIEF DESCRIPTION OF THE DRAWINGS

Figure 1a depicts a flow cytometric analysis of the binding of biotinylated β-subunit of pertussis toxin (PT) to human peripheral blood leukocytes (PBL's) expressing the CD2 marker (E-rosetting T cells).

Figure 1b depicts flow cytometric analysis of the binding of biotinylated β-subunit of pertussis toxin (PT) to human PBL's expressing the CD11b marker (granulocytes/ monocytes).

Figure 1c depicts a flow cytometric analysis of the binding of biotinylated β-subunit of pertussis toxin (PT) to human PBL's expressing the CD19 marker (B cells).

Figure 2a depicts flow cytometric analysis of the binding of biotinylated Sambucus nigra agglutinin (SNA) to human PBL's expressing the CD2 marker (E-rosetting T cells).

Figure 2b depicts flow cytometric analysis of the binding of biotinylated SNA to human PBL's expressing the CD11b marker (granulocytes/monocytes).

Figure 2c depicts flow cytometric analysis of the binding of biotinylated SNA to human PBL's expressing the CD19 marker (B cells).

Figure 3a depicts flow cytometric analysis of the binding of biotinylated Maackia amurensis agglutinin (MAA) to human PBL's expressing the CD2 marker (E-rosetting T cells).

Figure 3b depicts flow cytometric analysis of the binding of biotinylated MAA to human PBL's expressing the CD11b marker (granulocytes/monocytes).

Figure 3c depicts flow cytometric analysis of the binding of biotinylated MAA to human PBL's expressing the CD19 marker (B cells).

Figure 4 depicts the dose dependent inhibition of binding of biotinylated lectins, specifically SNA and MAA on tumor cell lines with Sialyl Lewis A Synsorb™ (Chembiomed Ltd).

Figure 5 depicts the results of a flow cytometric assay studying the binding of biotinylated SNA to human PBL's which have been activated with phytohemagglutinin (PHA).

Figure 6 depicts the DTH inflammatory responses as determined by footpad swelling, observed in groups of Balb/c mice which were immunized with Super Carrier® (SC) antigen, followed by the administration of SNA, the β-oligomer of pertussis toxin (PT), phosphate buffered saline (PBS) or no immunization.

Figure 7 depicts the DTH inflammatory responses, as determined by footpad swelling, observed in groups of Balb/c mice which were immunized with SC and then administered various dosages of SNA, PBS, or not immunized and challenged with the SC antigen.

Figure 8 depicts the development of the DTH inflammatory responses, as determined by footpad swelling, in groups of Balb/c mice which were administered SC in combination with SNA.

Figure 9 depicts the development of the DTH response, as determined by footpad swelling, in groups of Balb/c mice which were administered SC in combination with the β-subunit of pertussis toxin.

Figure 10 depicts the long term effects on the DTH responses determined by footpad swelling, in groups of Balb/c mice which are immunized with SNA, the β-oligomer of pertussis toxin (PT), PBS, or not immunized and then rechallenged with the SC antigen after 4 weeks.

Figure 11a examines the effect that various carbohydrate binding proteins have on the adhesion of human tumor cells (U937 cells) to human umbilical vein endothelial cells (HUVEC's).

Figure 11b examines the effect that various carbohydrate binding proteins have on the adhesion of polymorphonuclear cells (PMN's) to human umbilical vein endothelial cells (HUVEC's).

Figure 12 depicts percent lung weight reduction in groups of Balb/c mice administered E. coli lipopolysaccharide (LPS) intranasally followed by the administration of SNA, pertussis toxin β-oligomer (PT), Sialyl Lewis A (SLeA), and Sialyl Lewis X (SleX).

Figure 13 depicts the footpad swelling response in groups of Balb/c mice immunized with OVA (albumin, chicken egg, SIGMA) and DDA (Dimethyloctadecylammonium Bromide, Kodak) in PBS followed by administration of SleX, neuraminidase, PBS, or no immunization.

Figure 14 depicts percent lung weight reduction in groups of Balb/c mice administered E. coli lipopolysaccharide (LPS) intranasally followed by the administration of neuraminidase, sulfatase, beta-glucuronidase, ARC199 and ARC200.

Figure 15 depicts granulocyte reduction in lung lavages in groups of Balb/c mice administered E. coli lipopolysaccharide (LPS) intranasally followed by the administration of neuraminidase, sulfatase, beta-glucuronidase, ARC199 and ARC200.

### DETAILED DESCRIPTION OF THE PREFERRED EMBODIMENTS

The present invention is directed to the discovery that certain carbohydrate binding proteins when administered to a mammal are effective in suppressing inflammatory responses, inducing tolerance to an antigen, modulating the induction of immune responses to antigens, and inhibiting or enhancing cell adhesion events, e.g., involved in metastasis of tumor cells.

### 1. Definitions

As used herein, the following terms have the meanings set forth below:

"Inflammatory response" or "inflammatory disorder" will refer to immune reactions involving specific and non-specific defense systems. A specific defense system reaction is a specific immune system reaction to an antigen. Examples of specific defense system reactions include antibody responses to antigens, such as viruses, allergens, and delayed-type hypersensitivity. A non-specific defense system reaction is an inflammatory response mediated by leukocytes generally incapable of immunological memory. Such cells include macrophages, eosinophils and neutrophils. Examples of non-specific reactions include the immediate swelling after a bee sting, and the collection of PMN leukocytes at sites of bacterial infection (e.g., pulmonary infiltrates in bacterial pneumonias and pus formation in abscesses).

Other "inflammatory responses" or "inflammatory disorders" within the scope of the present invention include, e.g., autoimmune disorders such as rheumatoid arthritis, lupus, multiple sclerosis, post-ischemic leukocyte mediated tissue damage (reperfusion injury), frost-bite injury or shock, acute leukocyte-mediated lung injury (ARDS), asthma, traumatic shock, septic shock, nephritis, and acute and chronic inflammation, including atopic dermatitis, psoriasis, and inflammatory bowel disease. Various platelet-mediated pathologies such as atherosclerosis and clotting are also included within the definition of "inflammatory responses" or "inflammatory disorders". In addition, "inflammatory responses" or "inflammatory disorders" may include the adhesion of circulating cancer cells, with specific examples including carcinoma of the colon and melanoma.

"Sialic acid" refers to N-acetylated 5-amino-3,5-dideoxy-D-glycero-D-galacto-nonulosonic acid ("Neu5Ac") and to derivatives thereof. The nomenclature describing derivatives of sialic acid derivatives herein is as set forth by Reuter et al³⁴.

Chemical modifications of saccharide units are well known in the art. For example, chemically modified sialic acid derivatives include 9-azido-Neu5Ac, 9-amino-Neu5Ac, 9-deoxy-Neu5Ac, 9-fluoro-Neu5Ac, 9-bromo-Neu5Ac, 8-deoxy-Neu5Ac, 8-epi-Neu5Ac, 7-deoxy-Neu5Ac, 7-epi-Neu5Ac, 7-8-bis-epi-Neu5Ac, 4-O-methyl-Neu5Ac, 4-N-acetyl-Neu5Ac, 4,7-di-deoxy-Neu5Ac, 4-uno-Neu5Ac, 3-hydroxy-Neu5Ac, 3-fluoro-Neu5Ac acid as well as 6-thio analogues of Neu5Ac are known in the art. Methods for preparing such sialic acid derivatives are taught in commonly assigned Docket No. 000475-005, U.S. Serial No. 07/889,017, filed on May 26, 1992, which application is incorporated by reference in its entirety.

"α-2,6 sialic acid structures" refers to molecules comprising terminal Neu5Acα(2,6)galactose sequences or derivatives thereof. Molecules containing α-2,6 sialic acid structures have been identified as comprising part of the putative receptor structure for selectins and other lectins.

"α-2,3 sialic acid structures" refers to molecules comprising terminal Neu5Acα(2,3)galactose sequences or derivatives thereof. Molecules comprising α-2,3 sialic acid structures have similarly been identified as comprising part of the putative receptor structures for selectins and other lectins.

"Carbohydrate binding protein" will refer to any protein of non-immune origin, in particular a lectin, or fragment or derivative thereof which is capable of binding to a carbohydrate structure comprised on the surface of mammalian cells. Generally, in the present application, "carbohydrate binding proteins" will refer to proteins which are capable of binding terminal sialic acid structures, in particular molecules which contain α-2,6 sialic acid structures and/or α-2,3 sialic acid structures, or derivatives thereof.

"Lectins" refer to carbohydrate binding proteins or derivative or fragment thereof of non-immune origin often obtained from plants which comprise two or more carbohydrate binding sites. These binding proteins typically comprise the ability to agglutinate cells and to precipitate complex carbohydrates. Lectins are classified based upon their carbohydrate binding specificity and are well known in the art.

"Acute respiratory distress syndrome" or "ARDS" refers to an inflammatory condition comprising leukocyte mediated lung injury.

"Reperfusion injury" refers to an inflammatory condition comprising leukocyte mediated tissue damage.

"Pertussis toxin" or "PT" in this application will refer to the β subunit or β oligomer of the pertussis toxin (PT) which is one of the virulence factors produced by Bordetella pertussis, the etiological agent of whooping cough. This protein binds to both α-2,6 sialic acid structures and α-2,3 sialic acid structures.

"Sambucus nigra agglutinin" or "SNA" refers to a hemagglutinin-type plant lectin obtained from elderberry bark which binds with high affinity to oligosaccharides containing terminal sialic acid α(2,6)-linked to galactose, but which does not bind to molecules containing terminal α-2,3 sialic acid structures, or fragments or derivatives thereof capable of binding α-2,6 sialic acid structures.

"Maackia amurensis agglutinin" or "MAA" refers to a plant leukoagglutinin type lectin comprised in the seeds of Maackia amurensis which binds to molecules containing α-2,3 sialic acid structures, or fragments or derivatives thereof which are capable of binding α-2,3 sialic acid structures.

"Neuraminidase" refers to an enzyme which is capable of hydrolyzing the galactose-N-acetylneuraminic acid bond at the terminus of oligosaccharide chains of glycoproteins and glycolipids, thereby liberating N-acetylneuraminic acid and which is capable of binding molecules comprising α-2,6 sialic acid structures, or fragments or derivatives thereof capable of binding α-2,6 sialic acid structures.

"Fucosidases" refers to any enzyme that cleaves fucose from oligosaccharide chains of glycoproteins and glycolipids, which is capable of binding molecules comprising α-2,6 sialic acid structures, or fragments or derivatives thereof capable of binding α-2,6 sialic acid structures.

"β-galactosidase" refers to an enzyme which breaks the GlcNAc backbone and which is capable of binding molecules comprising α-2,6 sialic acid structures, or fragments or derivatives thereof capable of binding α-2,6 sialic acid structures.

"Sulfatases" refer to enzymes which cleave sulfate groups from carbohydrates.

"Cell-mediated immune response" refers to those mammalian immune responses which are mediated by cell-cell interactions. Included within this term are cell-mediated inflammatory responses to an antigen such as delayed-type hypersensitivity (DTH) responses as well as cell-mediated inflammatory responses arising from myocardial infarction, virus-induced pneumonia, shock and sequelae (e.g., multiple organ failure), acute respiratory distress syndrome, (ARDS), allergic responses and the like. Generally, the cell-mediated immune response is a leukocyte-mediated response.

"Humoral immune response" refers to mammalian immune responses which involve antigen-antibody interactions.

"DTH inflammation response" or delayed type hypersensitivity response is a T cell mediated reaction which results in a mononuclear cell-rich inflammation and swelling which occurs after antigenic challenge.

"Tolerance" or "Immunological tolerance" refers to a reduced immunogenic response elicited in a mammal to a particular antigen upon a second or subsequent antigenic challenge in comparison to the primary immune response elicited by said antigen under equivalent conditions (e.g., dosage). In the present invention such "tolerance" will be obtained by administration of an antigen followed by administration of one or more carbohydrate binding protein which bind to α-2,6 sialic acid structures and/or α-2,3 sialic acid structures.

### 2. Utility

Without being limited to any theory, it is believed that the subject carbohydrate binding proteins affect the immune response in a number of ways. Carbohydrate binding proteins can inhibit a mammal from becoming "educated" about a specific antigen when the carbohydrate binding protein is administered simultaneously with the first exposure of the immune system to the antigen. Also, carbohydrate binding proteins can inhibit the effector phase of a cell-mediated immune response (e.g., the inflammatory component of a DTH response) when administered after second or later exposures of the immune system to the antigen. Additionally, the subject carbohydrate binding proteins can induce tolerance to antigens when administered at the time of second or later exposures of the immune system to the antigen.

Further, the administration of carbohydrate binding proteins or fragments or derivatives thereof that bind α-2,6 sialic acid structures, and which may further bind to α-2,3 structures may affect the binding of LEC-CAM proteins and other lectins to the putative receptors therefor which include α-2,6 and α-2,3 structures.

The subject invention provides, in particular, a method for enhancing or inhibiting specific immune responses or cellular interactions in mammals by the administration of carbohydrate binding proteins of non-immune origin, e.g., lectins, or fragments or derivatives thereof capable of binding to α-2,6 and/or α-2,3 sialic acid structures.

Proteins, e.g., lectins, capable of binding α-2,6 and/or α-2,3 sialic acid structures are known in the art. Proteins capable of binding α-2,6 and/or α-2,3 sialic acid structures include, e.g., the β-subunit of pertussis toxin (PT), Sambucus nigra agglutinin (SNA), neuraminidases, sulfatases, fucosidases, Maackia amurensis agglutinin (MAA), β-galactosidases, and fragments or derivatives thereof capable of binding α-2,6 sialic acid structures and/or α-2,3 sialic acid structures.

However, the invention is not restricted to the use of the specifically exemplified carbohydrate binding proteins, but rather embraces the use of any fragment or derivative thereof which binds α-2,6 and/or α-2,3 sialic acid structures or molecules comprising such structures, which when administered to a mammal results in the enhancement or inhibition of immune responses and cellular interaction, in particular, inflammatory responses or conditions, tolerance to antigens, modulation of the immunogenic response to antigens, and the inhibition or enhancement of cell adhesion events, which are involved, e.g., in metastasis and inflammation.

It is well within the level of ordinary skill to identify other proteins capable of binding α-2,6 sialic acid structures and/or α-2,3 sialic acid structures, by conventional methods for assaying binding between ligands. Such methods include, e.g., competitive binding assays and receptor binding assays. The subject application, in particular, determines carbohydrate binding specificity utilizing the binding assay described in Pearce-Pratt et al³¹.

The subject invention accordingly further provides a method by which proteins capable of inducing or suppressing various immune responses and cellular interactions, e.g., inflammation, antigenic tolerance, modulation of antigenic response, or cell adhesion, may be putatively identified on the basis of their ability to bind α-2,6 and/or α-2,3 sialic acid structures.

Suitable proteins for use in the invention will be capable of binding α-2,6 sialic acid structures and/or α-2,3 sialic acid structures. However, an additional prerequisite of efficacious proteins will include suitability for in vivo administration. In particular, the carbohydrate binding protein should not be toxic, and should be sufficiently soluble at the required dosages, which will typically range from about 0.5-50 mg/kg of body weight.

In this regard, one of the carbohydrate binding compounds tested, specifically MAA, was found to be toxic. However, it may be possible to make derivatives of MAA, e.g., by chemical derivatization, mutagenesis, or by recombinant methods, which are not toxic and which are still capable of binding α-2,3 sialic acid structures. Thus, the invention further contemplates fragments or derivatives of proteins, e.g., lectins, capable of binding α-2,6 sialic acid and/or α-2,3 sialic structures which have been modified to render them non-toxic while still retaining the ability to bind α-2,6 sialic acid structures and/or α-2,3 sialic structures.

Similarly, carbohydrate binding proteins which are insufficiently soluble at the required dosages may be rendered soluble, e.g., by attachment to hydrophilic moieties, or by mutagenesis. Methods for solubilizing proteins are known in the art.

The subject invention provides, in particular, methods for suppressing inflammatory responses or disorders by the administration of an anti-inflammatory effective amount of one or more proteins, e.g., lectins, capable of binding α-2,6 and/or α-2,3 sialic acid structures or molecules comprising such sialic acid structures.

The inflammatory responses or disorders treatable by the subject invention include inflammatory immune reactions involving specific and non-specific defense systems. As discussed above, such conditions include antibody responses to antigens, such as viruses, allergens, delayed-type hypersensitivity, autoimmune disorders such as rheumatoid arthritis and lupus, post-ischemic leukocyte mediated tissue damage (reperfusion injury), frost-bite injury or shock-acute leukocyte-mediated lung injury (e.g., acute respiratory distress syndrome), asthma, traumatic shock, septic shock, nephritis, and acute and chronic inflammation, including atopic dermatitis, psoriasis, and inflammatory bowel disease. Further, inflammatory disorders treatable by the subject invention may include platelet-mediated pathologies such are atherosclerosis and clotting disorders.

Inflammatory conditions of special interest include delayed type hypersensitivity reactions, reperfusion, and acute leukocyte-mediated lung injury (ARDS).

The invention provides a generic method by which inflammatory responses or disorders may be suppressed by the administration of an effective amount of one or more proteins, e.g., lectins, or fragments or derivatives thereof capable of binding α-2,6 and/or α-2,3 sialic acid structures. However, in particular, the invention provides methods by which inflammatory responses or disorders may be treated or suppressed by the administration of an effective amount of one or more proteins selected from the β-subunit of pertussis toxin (PT), Sambucus nigra agglutinin (SNA), non-toxic derivatives of Maackia amurensis, fucosidases, β-galactosidases, neuraminidases, and derivatives or fragments thereof capable of binding α-2,6 sialic acid structures and/or α-2,3 sialic acid structures.

The subject invention further provides a general method for stimulating or inhibiting immune responses and cell adhesion events in mammals by the administration of an effective amount of one or more proteins or fragments or derivatives therof capable of binding α-2,6 and/or α-2,3 sialic acid structures.

Such immune responses include cell mediated and humoral immune responses. As has been discussed, such immune responses include, in particular, inflammatory responses or inflammatory disorders. The invention further provides methods for affecting the induction of immune responses to antigens comprising administering to a mammal an antigen in conjunction with one or more carbohydrate binding proteins, e.g, lectins, capable of binding α-2,6 sialic acid structures and/or α-2,3 sialic acid structures.

For example, it has been found that SNA when administered with an antigen modulates the induction of the immune response to the antigen. Accordingly, the subject carbohydrate binding proteins may comprise applicability as immune modulators, which may be administered in conjunction with vaccines, artificial organs or tissue transplants, and allogeneic organ and tissue transplants as a means for modulating the immune response to foreign antigens comprised therein.

It has further been found that the subject α-2,6 and/or α-2,3 binding proteins, when administered to a mammal which has been immunized with a particular antigen, may result in the induction of long term tolerance to said antigen.

In particular, it has been found that administration of SNA to mammals that have been immunized with an antigen, results in said mammals exhibiting a reduced immune response upon subsequent challenge(s) with said antigen. Thus, the subject carbohydrate binding proteins, in particular, SNA, have applicability as tolerogens. Given this property, such carbohydrate binding proteins or fragments or derivatives thereof, e.g., SNA, may be especially suitable in the treatment of allergic disorders since administration of "tolerogenic" derivatized allergens is a known means for treating allergic disorders.

The subject invention further provides methods for inhibiting or enhancing the adhesion of certain cell types, in particular, tumor cells and polymorphonuclear cells (PMN's) to endothelial cells. In this regard, it has been found that the SNA protein enhances in vitro binding of PMNs and tumor cells to endothelial cells which express E-selectin on their surface (ELAM-1).

In contrast, the β-oligomer of pertussis toxin (PT) inhibits the binding of PMN's and tumor cells to endothelial cells expressing ELAM-1. Tumor metastasis is thought to involve tumor cell adhesion to selectin bearing cells. Therefore, administration of α-2,6 and/or α-2,3 sialic acid structure binding proteins, to mammals should provide a method for inhibiting metastasis. For example, the subject carbohydrate binding proteins or fragments or derivatives thereof may be administered before, during or after cancer surgery or biopsy as a means for inhibiting metastasis of tumor cells which may be released into the circulatory system during surgery.

In the methods pertaining to suppression of inflammatory reactions or disorders, the subject carbohydrate binding proteins or fragments or derivatives thereof will generally be administered 1-15 hours after onset of the inflammatory response, and preferably about 1-10 hours after onset of inflammation.

In the methods pertaining to modulating the induction of an immune response to an antigen, the subject carbohydrate binding proteins or fragments or derivatives thereof will be administered in conjunction with the antigen.

In the methods pertaining to induction of long term tolerance to an antigen the subject carbohydrate binding proteins or fragments or derivatives thereof will generally be administered to a mammal which has been immunized with an antigen. Preferably, the carbohydrate binding protein or fragments or derivatives thereof will be administered about 1-15 hours after antigen exposure, and more preferably 1-10 hours after antigen exposure. However, these times may vary dependent upon the particular antigen and the carbohydrate binding protein which is administered.

In the methods pertaining to inhibiting metastasis the subject carbohydrate binding proteins or fragments or derivatives thereof will generally be administered at a time ranging from about 5 hours before cancer surgery or biopsy to about 15 hours after cancer surgery or biopsy, or during the cancer biopsy or surgery.

Generally, the subject carbohydrate binding proteins or fragments or derivatives thereof will be administered parenterally, e.g., by intramuscular or intravenous routes. However, other dosage forms should also be suitable including, e.g., oral, transdermal, rectal, intratracheal, and intranasal formulations. For example, intranasal and intratracheal formulations may be preferred if the inflammatory condition treated involves lung inflammation, e.g., acute respiratory distress syndrome (ARDS). In contrast, an oral formulation would likely be preferred if the inflammatory condition treated involves the digestive tract, e.g., inflammatory bowel disease.

Pharmaceutical compositions for use in the subject invention will generally comprise an effective amount of one or more proteins or fragments or derivatives thereof capable of binding α-2,6 sialic acid structures and/or α-2,3 sialic acid structures in combination with a pharmaceutically acceptable carrier and/or excipients. The particular pharmaceutically acceptable carrier and excipients will vary dependent upon the dosage form.

For example, parenteral dosage forms may contain phosphate buffered saline as a carrier, while intranasal formulations will comprise inhalants, and oral dosage forms may comprise enteric coatings. The selection of suitable carriers and excipients and formulation of different dosage forms is well within the level of ordinary skill in the pharmaceutical art.

The subject carbohydrate binding proteins or fragments or derivatives thereof will preferably be administered at dosages ranging from about 0.5 to 50mg/kg body weight, with 5-10 mg/kg being most preferred. Generally, the methods of the present invention will involve administration of a single dose of the subject carbohydrate binding proteins. However, the invention further contemplates repeated administration of the subject carbohydrate binding proteins or fragments or derivatives thereof. Repeated administration of the subject carbohydrate binding proteins may be desirable, e.g., in the treatment of chronic or sustained inflammatory disorders, such as, rheumatoid arthritis, acute and chronic inflammation, psoriasis, inflammatory bowel disorders, and autoimmune disorders associated with inflammatory responses, such as lupus, multiple sclerosis or rheumatoid arthritis.

### 3. Examples

In order to fully illustrate the present invention and the advantages thereof, the following specific examples are given, it being understood that these examples are intended to be illustrative only and in nowise limitative of the scope of the present invention.

Examples 1-11 illustrate the carbohydrate binding properties, anti-inflammatory properties, and immuno-regulatory properties of several carbohydrate binding-proteins. In these examples, the proteins employed are the B-subunit of pertussis toxin, the Sambucus nigra lectin isolated from the bark of elderberry trees, the lectin from Maackia amurensis, and neuraminidase.

### Example 1 -- Carbohydrate binding specificities of the B-subunit of pertussis toxin, the Sambucus nigra agglutinin and the Maackia amurensis agglutinin.

B-subunit of pertussis toxin (PT) was biotinylated as described previously³⁰ , while the biotinylated Sambucus nigra agglutinin (SNA) and the biotinylated Maackia amurensis agglutinin (MAA) were obtained commercially (Boehringer mannheim). Binding assays were carried out as reported earlier³¹ . Briefly, microtiter wells were coated with 50µl of BSA conjugates (50µg/ml) in 50 mM sodium phosphate buffer (pH 6.8) containing 5 mM MgCl₂ and 15 mM NaN₃ for 16 hours at 4°C. The solution was removed by aspiration and replaced with 100µl of 1 percent BSA in PBS containing 0.05 percent Tween 20 (PBST). After incubation for 2-3 hours at room temperature (RT), the microtiter wells were washed four times with 300µl of PBST. PT β-subunit-biotin, SNA-biotin or MAA-biotin (1/200 dilution) was then added to the micro-titer wells. After incubation for 1 hour at RT the plate was washed with PBST 4 times. Avidin-peroxidase (100µl of a 1/3000 dilution of a 1mg/ml solution in PBST) was added and an enzyme substrate solution consisting of 1mM ABTS in 5mM citrate buffer, pH4.2, containing 0.1 percent H₂O₂ v/v was added to the plate. Color development was allowed to occur for 30 minutes and was then measured at 405nm in a Titertek Multiskan ™ plate reader. The results are described in Tables 1-3 and the binding is expressed as a percentage relative to either the backbone structure αNeuAc(2-3)βGal(1-4)βGlcNAc-BSA or to αNeuAc(2-6)βGal(1-4)βGlcNAc-BSA. It can be seen in Table 1 that the PT β-oligomer-biotin binds to both Sialyl Lewis x and Sialyl Lewis A. More interesting is the observation that the PT β-oligomer-biotin binds with strongest affinity to the αNeuAc(2-6) βGal (1-4) βGlcNAC-BSA backbone.

**Table 1**

| **Binding of PT β Oligomer-biotin to BSA** | | |
|---|---|---|
| **Conjugates******* **Carbohydrate Structure of BSA Conjugate** | **A 405(X 1000)** | **% Binding Relative to BSA 123** |
| αNeuAc(2-3)βGal(1-4)βGlcNAc-BSA | 164± | 100 |
| αNeuAc(2-3)βGal(1-3)βGlcNAc-BSA | 163±4 | 99 |
| αNeuAc(2-3)βGal(1-4)βGlcNAc-BSA (1-3) αFuc | 143±8 | 87 |
| αNeuAc(2-3)βGal(1-3)βGlcNAc-BSA (1-4) αFuc | 158±12 | 96 |
| αNeuAc(2-6)βGal(1-3)βGlcNAc-BSA | 362±2 | 180 |
| αNeuAc(2-6)βGal(1-4)βGlcNAc-BSA | 653±23 | 398 |

| | | |
|---|---|---|
| * Experiments were done by coating µg/mL BSA conjugate and probed with 0.05 µg PT-β-biotin for 1 hour at room temperature. | | |

**Table 2**

| **Binding of MAA-biotin to BSA Conjugates******* | | |
|---|---|---|
| **Carbohydrate Structure of BSA Conjugate** | **A 405(x 1000)** | **% Binding Relative to BSA 123** |
| αNeuAc(2-3)βGal(1-4)βGlcNAc-BSA | 1379±59 | 100 |
| αNeuAc(2-3)βGal(1-3)βGlcNAc-BSA | 148±15 | 11 |
| αNeuAc(2-3)βGal(1-4)βGlcNAc-BSA (1-3) αFuc | 116±7 | 8 |
| αNeuAc(2-3)βGal(1-3)βGlcNAc-BSA (1-4) αFuc | 156±5 | 11 |

| | | |
|---|---|---|
| * Experiments were done by coating 50 µg/mL BSA-conjugate and probed with 0.25 µg MAA-biotin for 1 hour at room temperature. | | |

**Table 3**

| **Binding of SNA-biotin to BSA Conjugates******* | | |
|---|---|---|
| **Carbohydrate Structure of BSA Conjugate** | **A 405(x 1000)** | **% Binding Relative to BSA 87** |
| αNeuAc(2-3)βGal(1-4)βGlcNAc-BSA | 90±10 | 6 |
| αNeuAc(2-3)βGal(1-3)βGlcNAc-BSA | 112±1 | 7 |
| αNeuAc(2-6)βGal(1-3)βGlcNAc-BSA | 685±23 | 44 |
| αNeuAc(2-6)βGal(1-4)βGlcNAc-BSA | 1567±41 | 100 |

| | | |
|---|---|---|
| * Experiments were done by coating 50 µg/mL BSA-conjugate and probed with 0.25 µg SNA-biotin for 1 hour at room temperature. | | |

### Example 2 -- Expression of carbohydrate binding domains on populations of human leukocytes.

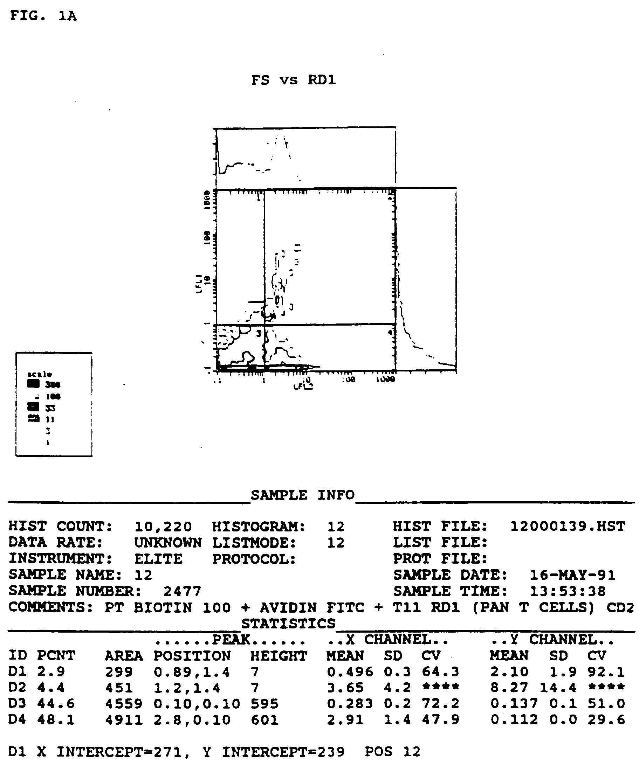

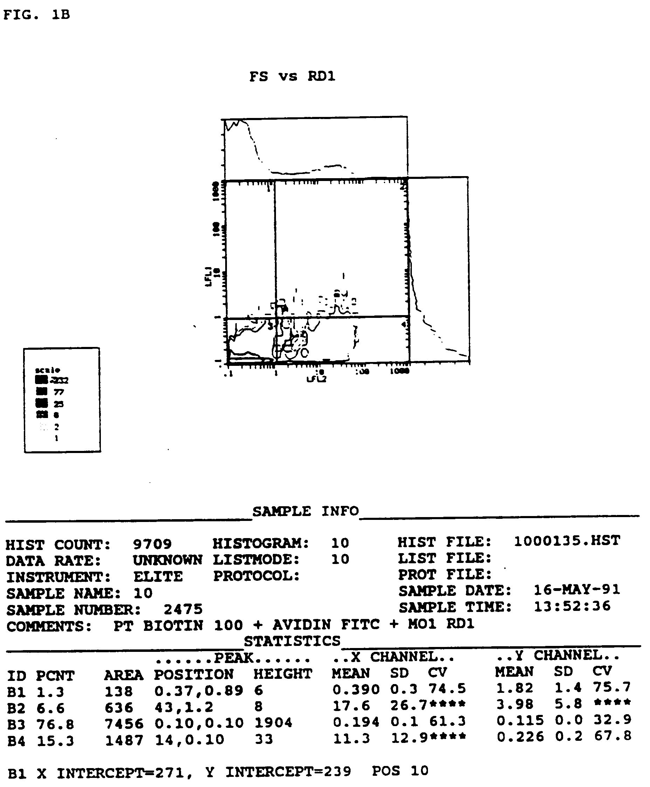

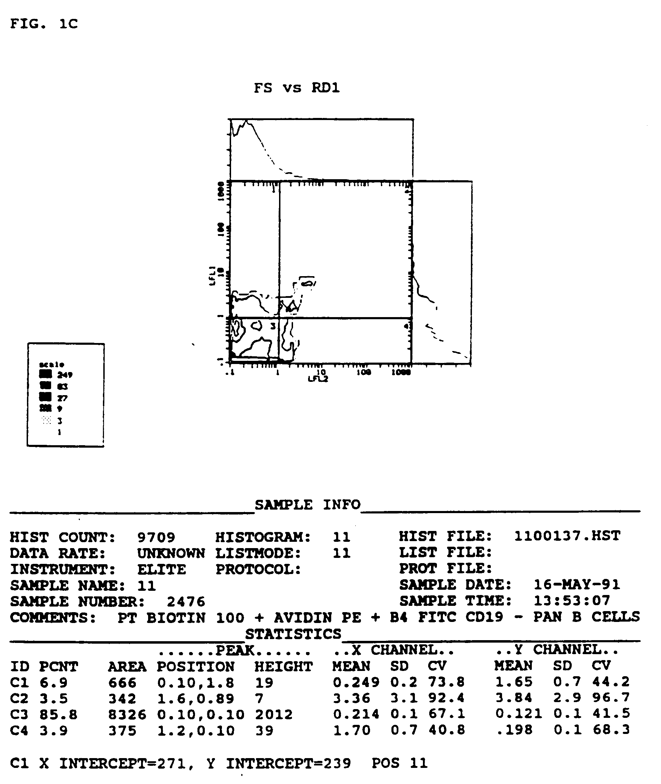

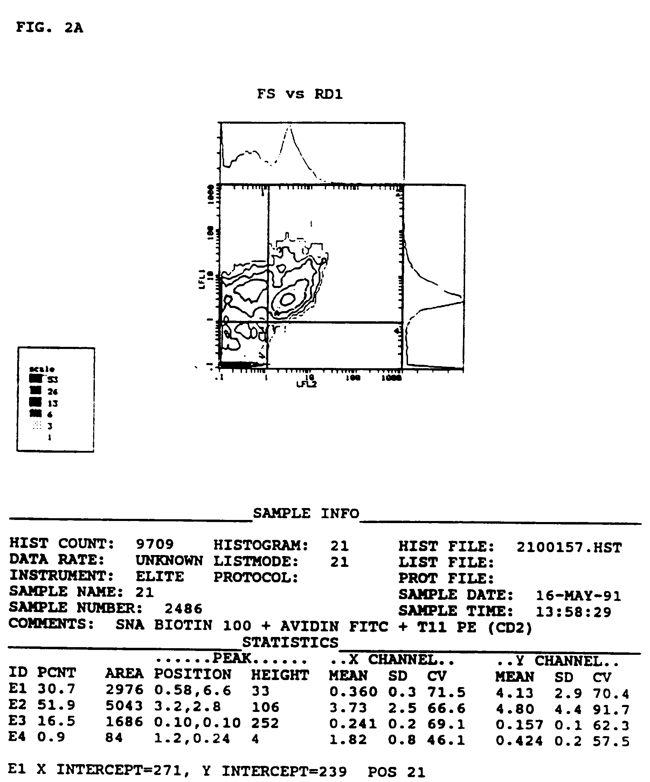

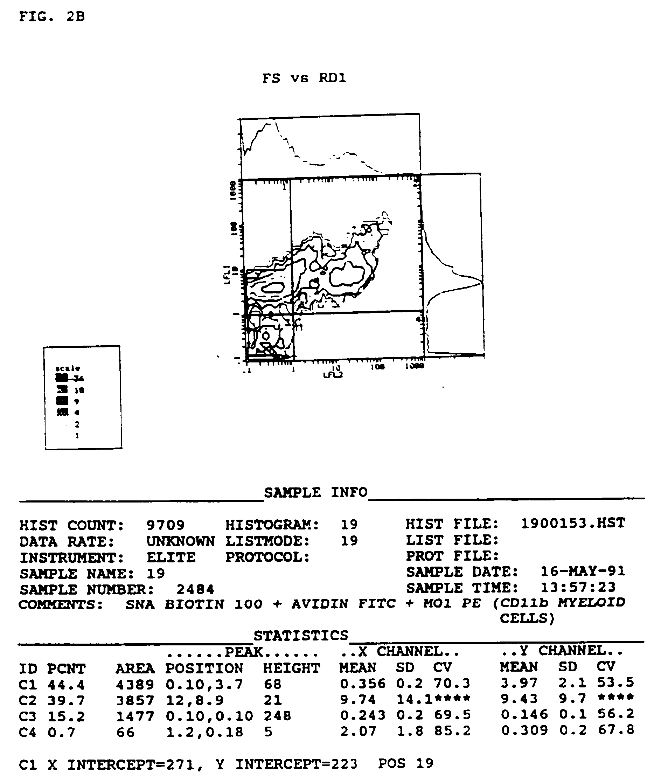

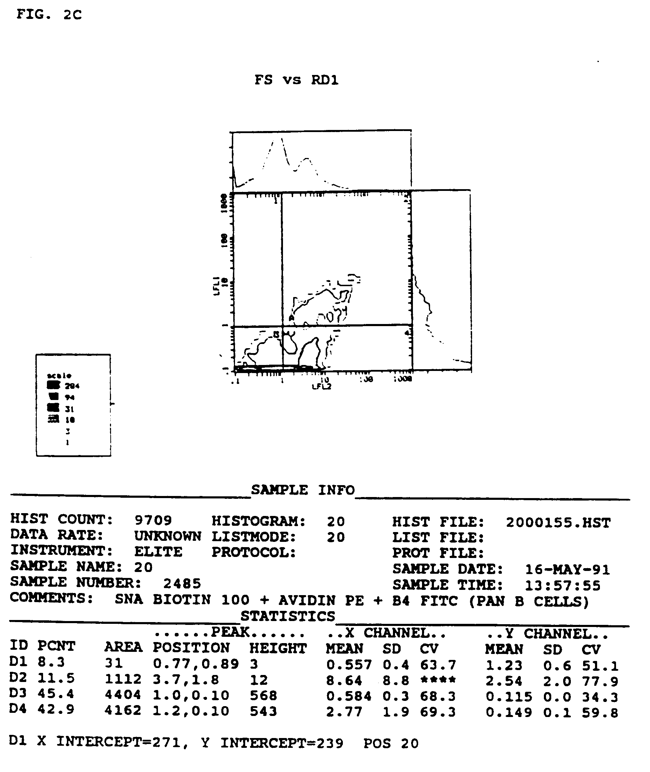

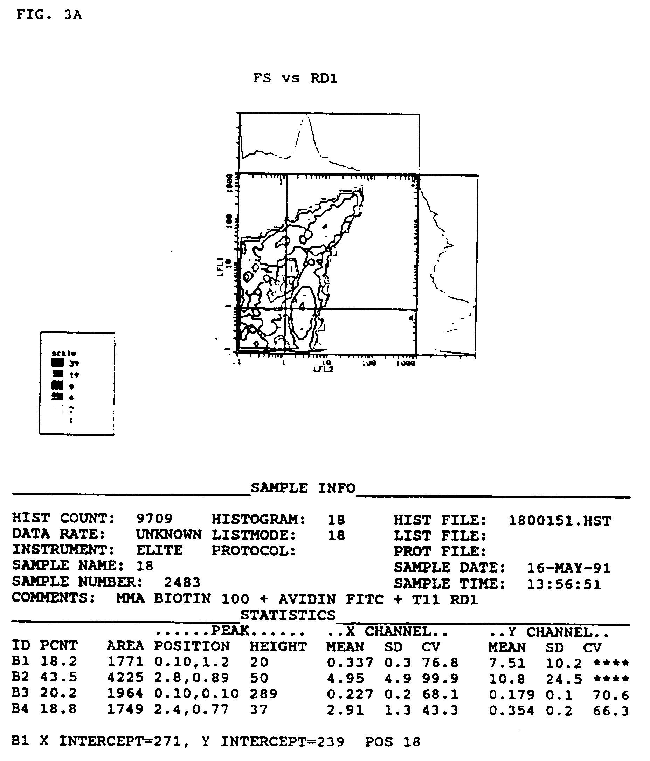

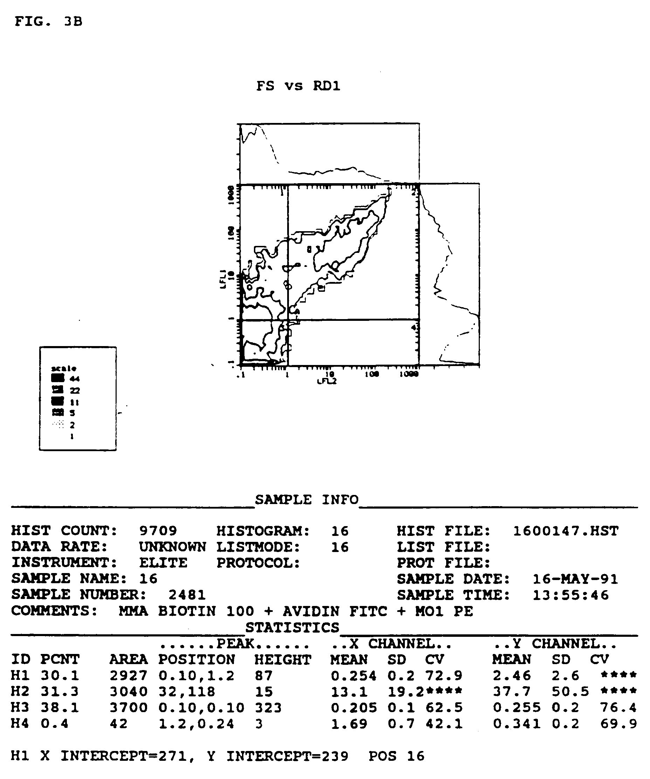

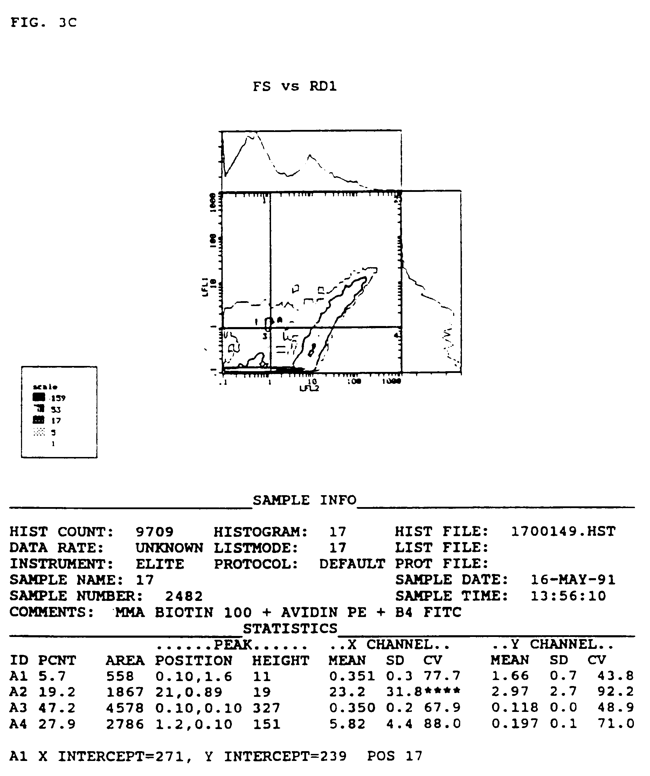

Peripheral blood leukocytes (PBL's) were obtained from normal volunteers. PBL's were aliquoted into samples containing 1x 10⁶ cells, these were then mixed with saturating concentrations of monoclonal antibodies labeled with a red fluorescent dye directed towards cell specific markers, and placed on ice for 1 hour. These cells were then washed and stained with biotinylated carbohydrate binding proteins. A green fluorescent labeled avidin which specifically binds to the biotinylated lectins was then added to the cell. The cells were analyzed on a flow cytometer (Coulter™ Profile II). The results of this experiment are shown in Figure 1-3. Figure 1 demonstrates that the biotinylated B-subunit of pertussis toxin does bind weakly to human PBL's, more specifically to cells expressing the CD2 marker (E-rosetting T Cells) 6.6 percent (Fig 1a), cells expressing CD11b marker (Granulocytes/monocytes) 4.4 percent (Fig 1b), and less to cells expressing the CD19 marker (B cells) 3.5 percent (Fig 1c). Figure 2 demonstrates that the biotinylated SNA does bind strongly to human PBL's, more specifically to cells expressing the CD2 marker (E-rosetting T Cells) 51.9 percent (Fig 2a), cells expressing CD11b marker (Granulocytes/monocytes) 39.7 percent (Fig 2b), and less to cells expressing the CD19 marker (B cells) 11.9 percent (Fig 2c). Figure 3 demonstrates that the biotinylated MAA does bind strongly to human PBL's, more specifically to cells expressing the CD2 marker (E-rosetting T Cells) 43.5 percent (Fig 3a), cells expressing CD11b marker (Granulocytes/monocytes) 31.3 percent (Fig 3b), and less to cells expressing the CD19 marker (B cells) 19.2 percent (Fig 3c).

### Example 3 -- Inhibition of Staining of labeled carbohydrate binding proteins by Sialyl Lewis A linked to synsorb.

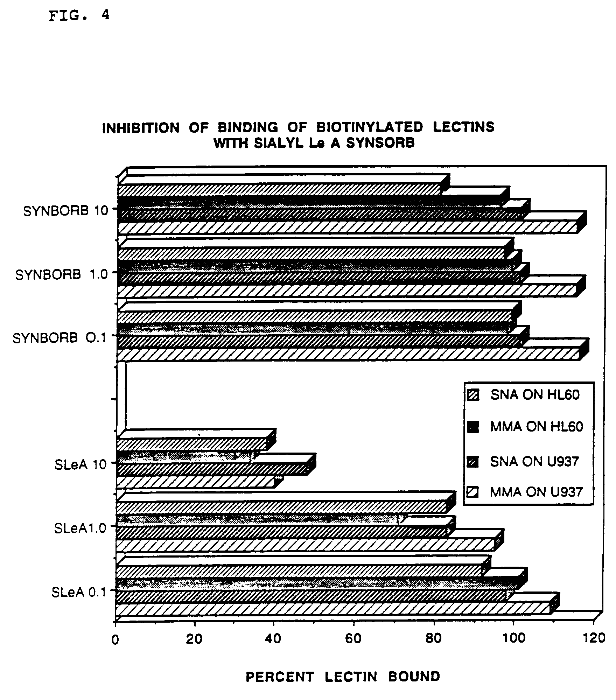

Cells from the tumor cell lines HL60 human and U937 mouse were stained for specific biotinylated lectins SNA and MAA as outlined in example 2. Aliquots of cells were also stained in the presence of the Sialyl Lewis A Synsorb™ (Chembiomed Ltd) or unlabeled Synsorb™ (Chembiomed Ltd). Figure 4 indicates that the Sialyl Lewis A Synsorb™ (Chembiomed LTD) can inhibit binding of both the SNA and MAA to both cell lines in a dose dependent manner. The results are expressed as a percentage of the control binding of the SNA and MAA. Thus, the results indicate that these carbohydrate binding proteins can bind to the carbohydrate structure Sialyl Lewis A.

### Example 4 -- Expression of carbohydrate binding domains on populations of human leukocytes before and after activation with PHA.

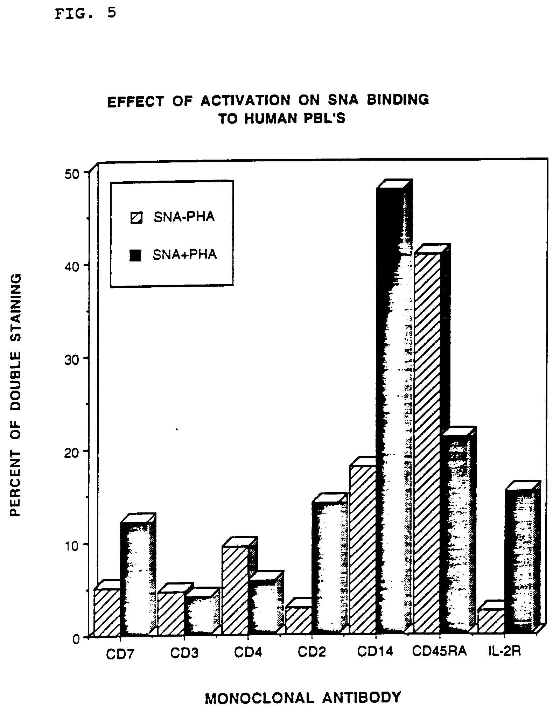

Peripheral blood leukocytes (PBL) were obtained from normal volunteers. PBL's were then cultured in RPMI 1640 (Gibco) supplemented with 10 percent AB serum in the presence or absence of PHA 10µg/ml at 37°C for 24 hours in 5 percent CO₂. Aliquots of stimulated and resting cells were then stained with saturating concentrations of monoclonal antibodies labeled with a red fluorescent dye-1 (RD-1) directed towards cell specific markers, and placed on ice for 1 hour. These cells were then washed and stained with biotinylated SNA. A green fluorescent labeled avidin which specifically binds to the biotinylated SNA was then added to the cells. These cells were then analyzed on a flow cytometer (Coulter™ Profile II). The results of this experiment demonstrate that the biotinylated SNA does bind to a variety of populations of human PBL's, and activation of these cells with PHA does affect the expression of carbohydrates with which SNA can bind (Fig. 5).

### Example 5 -- Inhibition of DTH Inflammatory Response.

DTH inflammatory responses were measured using the mouse footpad swelling assay as described by Smith and Ziola³² . Briefly, groups of Balb/c mice were immunized with 10 µg of the Super Carrier® (Pierce Rockford, Il. USA 61105) which has been shown to induce a strong mammalian inflammatory DTH response. Seven days later, each group of mice was footpad-challenged with 10 or 20 µg of the Super Carrier®. The resulting inflammatory footpad swelling was measured with a Mitutoyo Engineering micrometer 24 hours after challenge.

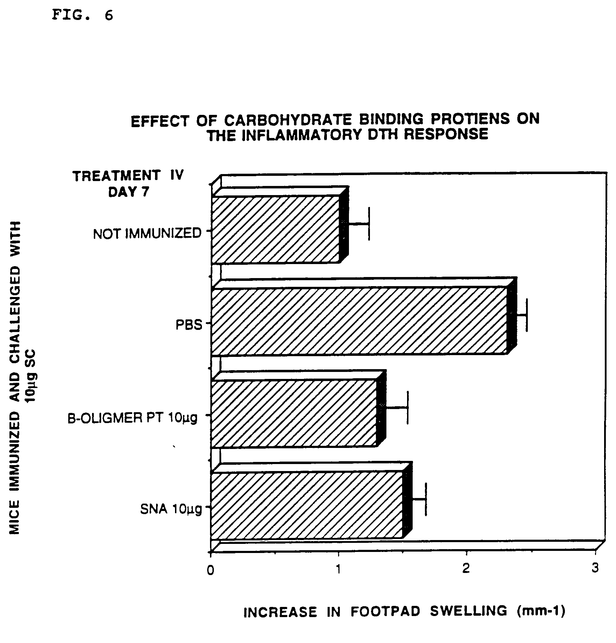

To assess the effect of the carbohydrate binding proteins on the inflammatory DTH response, groups of mice received 10 µg of protein, injected into the tail vein, 5 hours after challenge on the footpad. Control groups were left untreated or received 100 µL of phosphate-buffered saline (PBS). The results of this experiment are shown in Figure 6. The β-subunit of pertussis toxin decreased the inflammation by 76 percent when compared to control mice, whereas SNA decreased the inflammation by 61.5 percent compared to the control mice.

### Example 6 -- Dose-Dependency of the Anti-Inflammatory Properties of the Carbohydrate Binding Protein SNA.

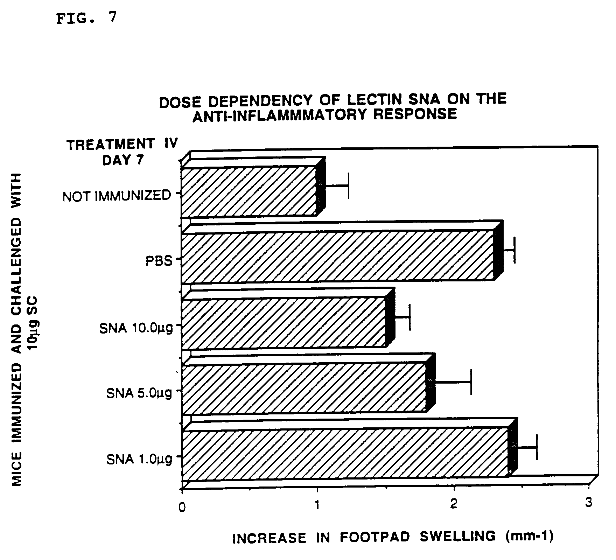

Four groups of mice were subjected to primary immunization and challenge with Super Carrier® (SC) as described under Example 5, above. Five hours after challenge on the footpad, groups were injected intravenously with 100µL solutions containing 0.0g, 1.0, 5.0, or 10.0 µg of the SNA in PBS. The DTH responses for each dose group were measured 24 hours after challenge and are shown in Figure 7. While the groups receiving PBS or 1.0 µg of lectin showed essentially the same extent of footpad swelling as positive controls, the groups receiving 5.0 or 10.0 µg of SNA displayed reduced footpad swelling 61% and 40% of the PBS controls, respectively.

### Example 7 -- Effect of the carbohydrate binding proteins on the induction of an immune response.

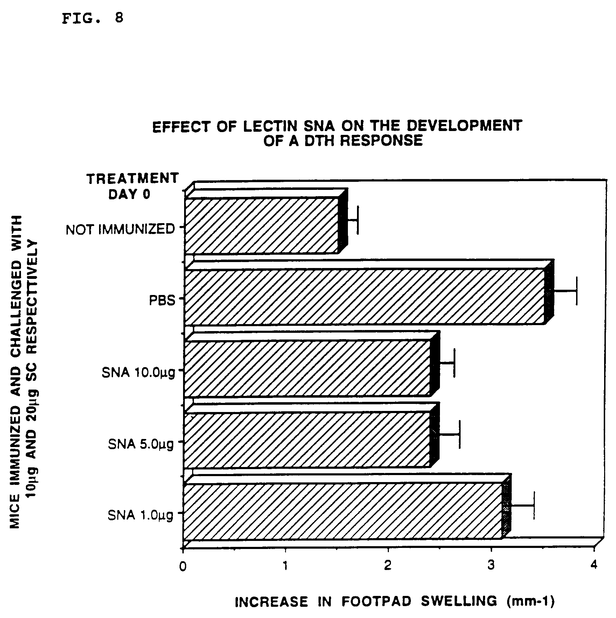

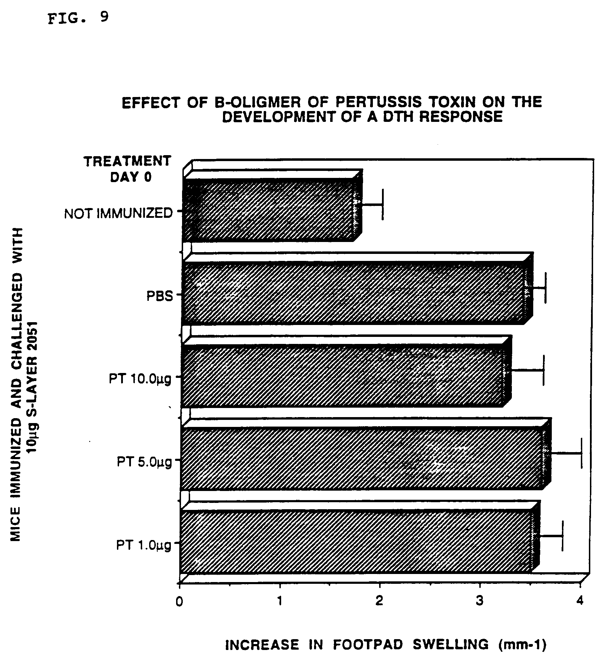

To examine the effect that the carbohydrate binding proteins have on the induction of an immune response, mice were immunized and challenged with SC as outlined in example 5, with the addition of either SNA or B-subunit of pertussis toxin at the time of immunization (before footpad challenge). Figure 8 demonstrates that the carbohydrate binding protein SNA does appear to have an ability to modulate the induction of an immune response. Figure 9, however, indicates the B-subunit of pertussis toxin does not possess this property and has no effect on the induction of an immune response to SC. This is despite the fact that work in the art has suggested that the whole pertussis toxin does have immuno-stimulatory properties³³.

### Example 8 -- Induction of a long term tolerance with carbohydrate binding proteins.

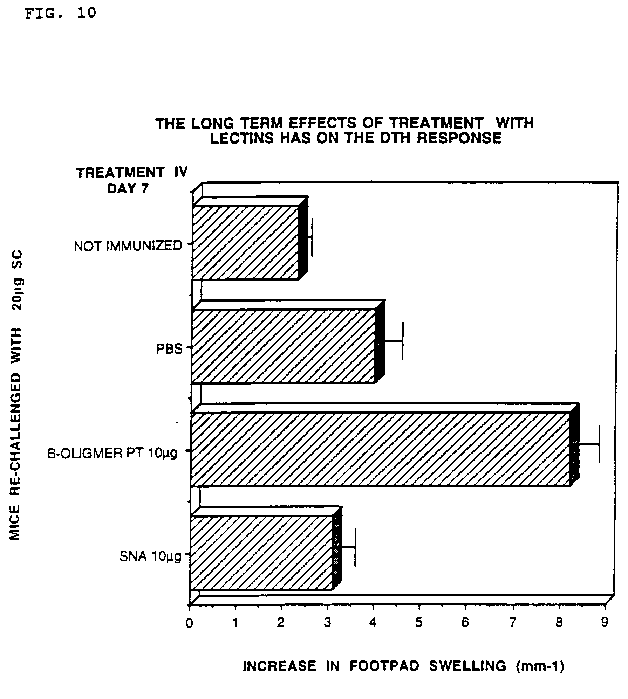

The identical groups of mice treated with the carbohydrate binding proteins in Example 5 were rechallenged with SC 4 weeks after primary immunization. Untreated controls responded with the usual degree of footpad swelling, whereas, the group treated with SNA showed reduced footpad swelling of 52 percent compared to the PBS controls (Fig 10). However the group of mice treated with the B-subunit of pertussis toxin had an augmented response greater than that of the controls by 100 percent.

In addition to providing suppression of cell-mediated immune responses, the above data demonstrate that treatment with the carbohydrate binding protein SNA as per this invention also imparts tolerance to additional challenges from the same antigen.

### Example 9 -- Effect of Carbohydrate Binding Proteins on ELAM-1 Dependent Cell Adhesion to Activated Vascular Endothelium

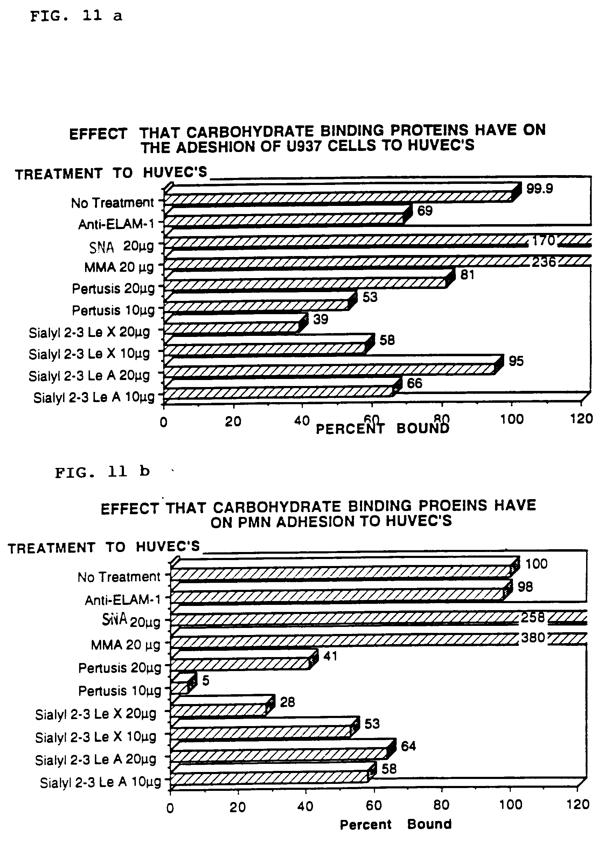

This example examines whether the carbohydrate binding proteins could inhibit ELAM-1 dependent cell adhesion to activated vascular endothelium. Specifically, an *in vitro* cell binding assay was performed as described by Lowe et al.¹⁸ . Briefly, human umbilical vein endothelial cells (HUVECs purchased from Cell Systems, Seattle, WA, USA) were stimulated with TNF (10 µg/ml) to express ELAM-1. Human tumor cell lines, human polymorphonuclear cells (PMN) or HL60, which have been shown to bind to HUVECs, in an ELAM-1 dependent manner were used to measure the effect that SNA has on the ELAM-1 dependent binding to HUVEC. Figures 11 a and b set forth the results of this example illustrating the effect that these compounds have on ELAM-1 dependent binding to the HUVECs. The B-oligomer of pertussis toxin inhibits binding of both PNM's and HL60's to HUVEC's. This is consistent with the published art. However the SNA augments binding of both the PMN's and the HL60's to the HUVEC's. Given this enhancement of binding it is postulated that SNA is a multi-functional lectin which functions as a cell cross-linking agent. This same type of activation has also been reported for antibodies directed towards the Sialyl Lewis X determinant on PMN's which enhance cell adhesion and yet are still able to block inflammation in vivo.

### Example 10 -- Effect of Carbohydrate Binding Proteins and Other Compounds on E. coli LPS Caused Lung Injury.

E. coli LPS (lipopolysaccharide) caused lung injury is measured by weighing the lungs of sacrificed mice 24 hours after the mice are given E. coli LPS intranasally. Specifically, groups of 8-10 week old Balb/c mice were sensitized with 5µg/mouse of E. coli LPS in 50µl of PBS intranasally under light anesthesia. Five hours later, 50µg/mouse of SNA, 100µg/mouse of SNA 10µg/mouse pertussis toxin β subunit, 20µg/mouse pertussis toxin β subunit, (List Biological Laboratories, Inc., USA), 200µg/mouse of SleX, or 200µg/mouse of SleA in 200µl of PBS were administered intravenously. After 24 hours, the mice were sacrificed and the lungs removed and weighed.

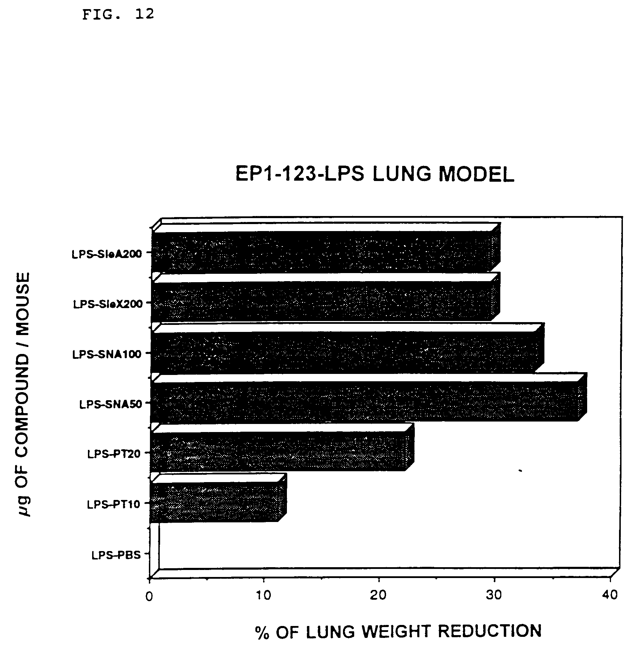

The results of this experiment are presented in Figure 12. These results indicate that the SNA compound provides about a 37% reduction in the DTH lung inflammatory response, and the pertussis toxin (PT) provides about a 22% reduction in the DTH inflammatory response. Administration of the SleA and SleX compounds resulted in about a 30% reduction in DTH inflammatory response.

Thus, these results indicate that the subject carbohydrate binding proteins should be suitable for reducing lung inflammation, in particular, lung inflammation caused by antigen exposure, such as Acute Respiratory Distress Syndrome (ARDS).

### Example 11 -- Effect of Neuraminidase Administration on OVA Induced DTH Inflammatory Response.

This example examines the effects of another α-2,6 sialic acid binding protein, neuraminidase, on the DTH inflammatory response induced by OVA (Albumin, chicken egg, SIGMA). DTH inflammatory responses were again measured using the footpad swelling assay as described by Smith et al³². In particular, groups of 10 of Balb/C mice, aged 8-10 weeks and weighing about 20-25 grams were immunized with 100µg of OVA (Albumin, chicken egg, SIGMA) and 20µg of DDA (Dimethyldioctadecylammonium Bromide, KODAK) in 100µl of PBS (phosphate buffered saline) per mouse, wherein administration was effected intramuscularly into the hind leg muscle. Seven days later, each group of mice was footpad challenged with 20µg of OVA comprised in 20µl of PBS. The resulting inflammatory footpad swelling was again measured with a Mitutoyo Engineering Micrometer.

To assess the effect of the subject carbohydrate binding proteins on the inflammatory DTH response, groups of mice received 100µg/mouse of SleX/mouse, 0.5 units/mouse of neuraminidase, or 1.0 units/mouse of neuraminidase in 200µl in PBS. Control groups were left untreated or received 200µl of phosphated buffered saline (PBS).

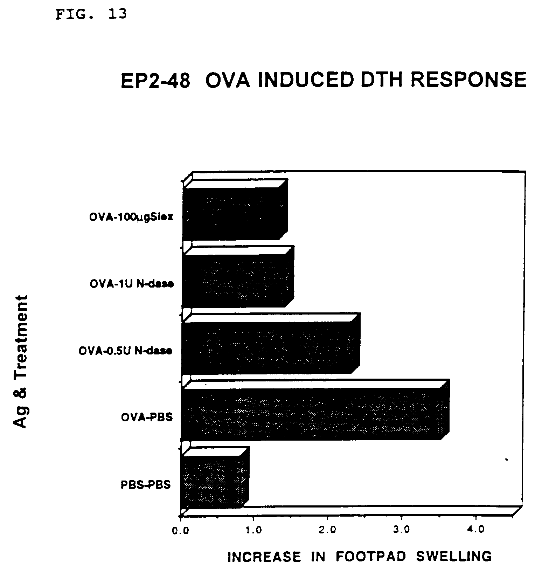

The results are depicted in Figure 13. In the groups of mice treated with neuraminidase, the DTH inflammation response was about 20% for the group administered 1.0 unit of neuraminidase, as compared to the control group, and about 55.6% for the group administered 0.5 unit of neuraminidase as compared to the control group.

Thus, these results indicate that the reduction in the DTH inflammatory response is proportional to the amount of neuraminidase administered, and that neuraminidase when administered in vivo inhibits DTH inflammatory responses induced by antigens.

### Example 12 -- Effect of Neuraminidase, Sulfatase, and Beta-Glucuronidase on E. coli LPS Caused Lung Injury.

E. coli LPS (lipopolysaccharide) caused lung injury is measured by weighing the lungs of sacrificed mice 24 hours after the mice are given E. coli LPS intranasally. Specifically, groups of 8-10 week old Balb/c mice were sensitized with 10µg/mouse of E. coli LPS in 50µl of PBS intranasally under light anesthesia. Four hours later, 100µg/mouse ARC 199, 100µg/mouse ARC 200, 0.5 U/mouse neuraminidase (type II, Sigma), 1.0 U/mouse sulfatase (type IV from limpets, Sigma) or 1.0 U/mouse beta-glucuronidase (type X-A from E. coli, Sigma) in 200µl of PBS were administered intravenously. After 24 hours, the mice were sacrificed and the lungs removed and weighed.

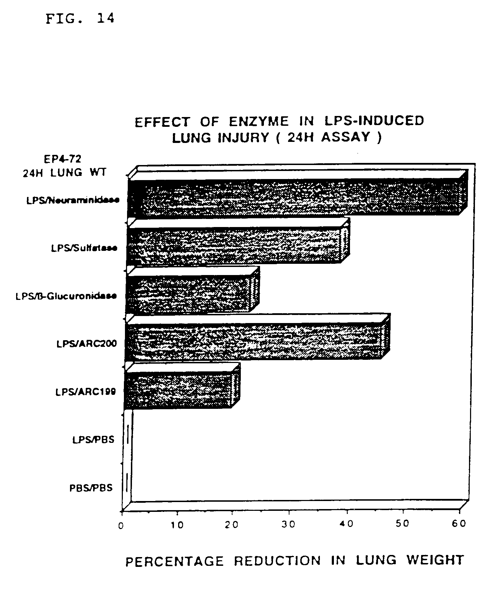

The results of this experiment are presented in Figure 14. These results indicate that neuraminidase provides about a 58% reduction in the DTH lung inflammatory response, sulfatase provides about a 40% reduction in the DTH lung inflammatory response, and beta-glucuronidase provides about a 22% reduction in the DTH inflammatory response. Administration of the ARC199 and ARC200 compounds resulted in about a 20% and 47% reduction, respectively, in DTH inflammatory response.

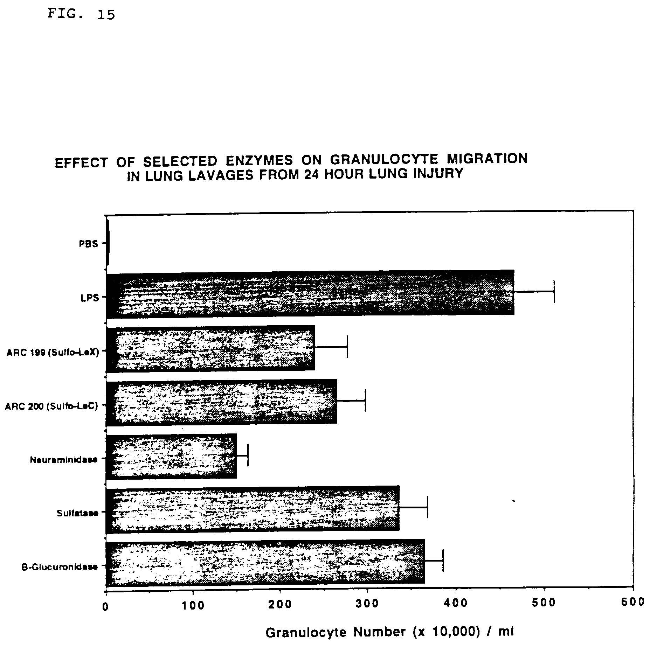

The effect of these enzymes on granulocyte migration in lung lavages from these mice is presented in Figure 15. Neuraminidase reduced granulocytes in lung lavages by about 67%, sulfatase reduced granulocytes by about 25%, and beta-glucuronidase reduced granulocytes by about 18%. Administration of the ARC199 and ARC200 compounds resulted in about a 50% and 45% reduction in granulocytes, respectively.

Thus, these results indicate that the subject enzymes should be suitable for reducing lung inflammation, in particular, lung inflammation caused by antigen exposure, such as Acute Respiratory Distress Syndrome (ARDS).

## Claims

1. Use of at least one carbohydrate binding protein or fragment or derivative thereof which binds α-2,6 sialic acid structures for the preparation of a pharmaceutical compostion for suppressing an inflammatory response in a mammal.

2. The use of claim 1, wherein said inflammatory response is associated with a delayed type hypersensitivity (DTH) inflammatory response, acute respiratory distress syndrome (ARDS), reperfusion injury or septic shock.

3. The use of claim 1, wherein the carbohydrate binding protein is selected from the group consisting of the pertussis toxin β-subunit (PT), Sambucus nigra agglutinin (SNA), neuraminidases, sulfatases, fucosidases, and β-galactosidases and fragments or derivatives thereof.

4. The use of claim 1, wherein said at least one carbohydrate binding protein or fragment or derivative thereof is also capable of binding α-2,3 sialic acid structures.

5. Use of at least one carbohydrate binding protein or fragment or derivative thereof which binds α-2,6 sialic acid structures for the preparation of a pharmaceutical composition for modulating the induction of an immune response to an antigen whereby the mammal is immunized with an antigen in combination with the protein.

6. The use of claim 5, wherein at least one of the carbohydrate binding proteins, or fragments or derivatives thereof is also capable of binding α-2,3 sialic acid structures.

7. The use of claim 5, wherein said immune response comprises a humoral or cell mediated immune response.

8. The use of claim 5, wherein the antigen comprises an allergen.

9. The use of claim 5, wherein the carbohydrate binding protein or fragment or derivative thereof comprises Sambucus nigra lectin (SNA).

10. The use of claim 5, wherein said at least one carbohydrate binding protein or fragment or derivative thereof is further capable of binding α-2,3 sialic acid structures.

11. Use of at least one carbohydrate binding protein or fragment or derivative thereof which binds α-2,6 sialic acid structures for the preparation of a pharmaceutical composition for inducing tolerance to an antigen in a mammal whereby the protein is administered to a mammal which has previously been exposed to an antigen.

12. The use of claim 11, wherein the antigen comprises an allergen.

13. The use of claim 11, wherein said at least one of said carbohydrate binding proteins or fragments or derivatives thereof is also capable of binding α-2,3 sialic acid structures.

14. The use of claim 11, wherein said carbohydrate binding protein is selected from the group consisting of the β-subunit of pertussis toxin (PT), Sambucus nigra agglutinin (SNA), neuraminidases, sulfatases, fucosidase and β-galactosidases, and fragments or derivatives thereof.

15. Use of at least one carbohydrate binding protein or fragment or derivative thereof capable of binding α-2,6 sialic acid structures for the preparation, of a pharmaceutical compostion for treating or inhibiting lung inflammation or a lung inflammatory disorder.

16. The use of claim 15, wherein the mammal comprises acute respiratory distress syndrome (ARDS).

17. The use of claim 15, wherein the carbohydrate binding protein or fragment or derivative thereof is selected from the group consisting of the β-subunit of pertussis toxin (PT), SNA, neuraminidases, sulfatases, fucosidases and β-galactosidases.

18. The use of claim 15, wherein the carbohydrate binding protein or fragment or derivative thereof is additionally capable of binding α-2,3 sialic acid structures.

19. Use of at least one carbohydrate binding protein or fragment or derivative thereof capable of binding α-2,6 sialic acid structures for the preparation of a pharmaceutical composition for treating or inhibiting metastasis.

20. The use of claim 19, wherein the carbohydrate binding protein or a fragment or derivative thereof is also capable of binding α-2,3 sialic acid structures.

21. The use of claim 19, wherein the mammal comprises colon carcinoma or melanoma.

## Patentansprüche

1. Verwendung mindestens eines Kohlenhydrat-bindenden Proteins, Fragmente oder Derivats davon, das an α-2,6 Sialinsäurestrukturen bindet zur Herstellung einer pharmazeutischen Zusammensetzung zur Unterdrückung von Entzündungsreaktionen in Säugern.

2. Verwendung nach Anspruch 1, wobei die Enzündungsreaktion mit einer Spättyp-Überempfindlichkeit (DTH)-Entzündungsreaktion aesoziiert ist, dem akuten Atemnotsyndrom (ARDS), Wiederdurchblutungsschäden oder einem septischen Schock.

3. Verwendung nach Anspruch 1, wobei das Kohlenhydratbindende Protein ausgewählt ist aus der Gruppe bestehend aus der Pertussistoxin β-Untereinheit (PT), Sambucus Nigra Agglutinin (SNA), Neuraminidasen, Sulfatasen, Fucosidasen und β-Galactosidasen und Fragmenten oder Derivaten davon.

4. Verwendung nach Anspruch 1, wobei mindestens ein Kohlenhydrat-bindendes Protein, Fragment oder Derivat davon auch an die α-2,3 Sialinsäurestrukturen binden kann.

5. Verwendung mindestens eines Kohlenhydrat-bindenden Proteine, Fragmente oder Derivate davon, das an α-2,6 Sialinsäurestrukturen bindet, zur Herstellung einer pharmazeutischen Zusammensetzung zum Regulieren der Induktion einer Immunantwort auf ein Antigen, wobei der Säuger mit einer Kombination eines Antigens mit dem Protein immunisiert wird.

6. Verwendung nach Anspruch 5, wobei mindestens eines der Kohlenhydat-bindenden Proteine, Fragmente oder Derivate davon auch an α-2,3 Sialinsäurestrukturen binden kann.

7. Verwendung nach Anspruch 5, wobei die Immunantwort eine humoral oder zellulär vermittelte Immunantwort umfasst.

8. Verwendung nach Anspruch 5, wobei das Antigen ein Allergen umfasst.

9. Verwendung nach Anspruch 5, wobei das Kohlenhydratbindende Protein, Fragment oder Derivat davon Sambucus Nigra Lectin (SNA) umfasst.

10. Verwendung nach Anspruch 5, wobei das mindestens eine Kohlenhydrat-bindende Protein, Fragment oder Derivat davon zudem an α-2,3 Sialinsäurestrukturen binden kann.

11. Verwendung mindestens eines Kohlenhydrat-bindenden Proteins, Fragments oder Derivats davon, das an α-2,6 Sialinsäurestrukturen bindet zur Herstellung einer pharmazeutischen Zusammensetzung zur Induzieren von Antigentoleranzen in Säugern, wobei das Protein einem Säuger verabreicht wird, der zuvor einem Antigen exponiert wurde.

12. Verwendung nach Anspruch 11, wobei das Antigen ein Allergen umfasst.

13. Verwendung nach Anspruch 11, wobei das mindestens eine Kohlenhydrat-bindende Protein, Fragment oder Derivat davon auch an α-2,3 Sialinsäurestrukuren binden kann.

14. Verwendung nach Anspruch 11, wobei das Kohlenhydrat-bindende Protein ausgewählt ist aus der Gruppe bestehend aus der β-Untereinheit von Petussistoxin (PT), Sambucus Nigra Agglutinin (SNA), Neuraminidasen, Sulfatasen, Fucosidasen und β-Galactosidasen und Fragmenten oder Derivaten davon.

15. Verwendung mindestens eines Kohlenhydrat-bindenden Proteins, Fragmente oder Derivats davon, das an α-2,6 Sialinsäurestrukturen binden kann zur Herstellung einer pharmazeutischen Zusammensetzung zur Behandlung oder Hemmung von Lungenentzündungen oder einem Lungenentzündungsleiden.

16. Verwendung nach Anspruch 15, wobei der Säuger ein akutes Atemnotsyndrom (ARDS) aufweist.

17. Verwendung nach Anspruch 15, wobei das Kohlenhydrat-bindende Protein, Fragment oder Derivat davon ausgewählt ist aus der Gruppe bestehend aus der β-Untereinheit von Pertussistoxin (PT), SNA, Neuraminidasen, Sulfatasen, Fucosidasen und β-Galactosidasen.

18. Verwendung nach Anspruch 15, wobei das Kohlenhydrat-bindende Protein, Fragment oder Derivat davon zudem an α-2,3 Sialinsäurestrukturen binden kann.

19. Verwendung mindestens eines Kohlenhydrat-bindenden Proteine, Fragments oder Derivate davon, das an α-2,6 Sialinsäurestrukturen binden kann zur Herstellung einer pharmazeutischen Zusammensetzung zur Behandlung oder Hemmung von Metastasen.

20. Verwendung nach Anspruch 19, wobei ein Kohlenhydrat-bindendes Protein, Fragment oder Derivat davon auch an α-2,3 Sialinsäurestrukturen binden kann.

21. Verwendung nach Anspruch 19, wobei der Säuger ein Coloncarcinom oder Melanom aufweist.

## Revendications

1. Utilisation d'au moins une protéine de liaison aux glucides ou d'un fragment ou d'un dérivé de cette dernière qui se lie à des structures α-2,6 d'acide sialique pour la préparaiion d'une composition pharmaceutique destinée à supprimer une réaction inflammatoire chez un mammifère.

2. Utilisation selon la revendication 1, où ladite réaction inflammatoire est associée à une réaction inflammatoire d'hypersensibilité de type retardé (DTH), à un syndrome de détresse respiratoire aigüe (ARDS), à une lésion de reperfusion ou à un choc septique.

3. Utilisation selon la revendication 1, où la protéine de liaison aux glucides est sélectionnée dans le groupe constitué par la sous-unité β de la toxine de la coqueluche (PT), l'agglutinine de Sambucus nigra (SNA), des neuraminidases, des sulfatases, des fucosidases et des β-galactosidases et leurs fragments ou leurs dérivés.

4. Utilisation selon la revendication 1, où ladite au moins une protéine de liaison aux glucides ou son fragment ou son dérivé est également capable de se lier à des structures α-2,3 d'acide sialique.

5. Utilisation d'au moins une protéine de liaison aux glucides ou d'un fragment ou d'un dérivé de cette dernière qui se lie à des structures α-2,6 d'acide sialique pour la préparation d'une composition pharmaceutique destinée à moduler l'induction d'une réponse immunitaire à un antigène, le mammifère étant immunisé au moyen d'un antigène associé à la protéine.

6. Utilisation selon la revendication 5, où au moins la protéine de liaison aux glucides et/ou son fragment et/ou son dérivé est également capable de se lier à des structures α-2,3 d'acide sialique.

7. Utilisation selon la revendication 5, où ladite réponse immunitaire comprend une réponse immunitaire à médiation humorale ou cellulaire.

8. Utilisation selon la revendication 5, où l'antigène comprend un allergène.

9. Utilisation selon la revendication 5, où la protéine de liaison aux glucides ou son fragment ou son dérivé comprend une lectine de Sambucus nigra (SNA).

10. Utilisation selon la revendication 5, où ladite au moins une protéine de liaison aux glucides ou son fragment ou son dérivé est en outre capable de se lier à des structures α-2,3 d'acide sialique.

11. Utilisation d'au moins une protéine de liaison aux glucides ou d'un fragment ou d'un dérivé de cette dernière qui se lie à des structures α-2,6 d'acide sialique pour la préparation d'une composition pharmaceutique destinée à induire une tolérance à un antigène chez un mammifère, la protéine étant administrée à un mammifère qui a préalablement été exposé à un antigène.

12. Utilisation selon la revendication 11, où l'antigène comprend un allergène.

13. Utilisation selon la revendication 11, où au moins ladite au moins une protéine de liaison aux glucides et/ou son fragment et/ou son dérivé est également capable de se lier à des structures α-2,3 d'acide sialique.

14. Utilisation selon la revendication 11, où ladite protéine de liaison aux glucides est sélectionnée dans le groupe constitué par la sous-unité β de la toxine de la coqueluche (PT), l'agglutinine de Sambucus nigra (SNA), des neuraminidases, des sulfatases, des fucosidases et des β-galactosidases et de leurs fragments ou dérivés.

15. Utilisation d'au moins une protéine de liaison aux glucides ou d'un fragment ou d'un dérivé de cette dernière capable de se lier à des structures α-2,6 d'acide sialique pour la préparation d'une composition pharmaceutique destinée à traiter ou inhiber l'inflammation pulmonaire ou un trouble inflammatoire pulmonaire.

16. Utilisation selon la revendication 15, où le mammifère présente un syndrome de détresse respiratoire aigüe (ARDS).

17. Utilisation selon la revendication 15, où la protéine de liaison aux glucides ou son fragment ou son dérivé est sélectionné dans le groupe constitué par la sous-unité β de la toxine de la coqueluche (PT), SNA, des neuraminidases, des sulfatases, des fucosidases et des β-galactosidases.

18. Utilisation selon la revendication 15, où la protéine de liaison aux glucides ou son fragment ou son dérivé est en outre capable de se lier à des structures α-2,3 d'acide sialique.

19. Utilisation d'au moins une protéine de liaison aux glucides ou d'un fragment ou d'un dérivé de cette dernière capable de se lier à des structures α-2,6 d'acide sialique pour la préparation d'une composition pharmaceutique destinée au traitement ou à l'inhibition de métastases.

20. Utilisation selon la revendication 19, où la protéine de liaison aux glucides ou son fragment ou son dérivé est également capable de se lier à des structures α-2,3 d'acide sialique.

21. Utilisation selon la revendication 19, où le mammifère présente un cancer colique ou un mélanome.
